# EUROPEAN PATENT SPECIFICATION

(11) **EP 1 002 789 B1**
(45) Date of publication and mention of the grant of the patent: **18.06.2003**
(21) Application number: 98309492.1
(22) Date of filing: 19.11.1998
(51) Int. Cl.: C07C 209/68, C07C 209/18

(54) **Preparation of alkylated aromatic amines**
Herstellung von alkylierten aromatischen Aminen
Préparation d'amines aromatiques alcoylées

(43) Date of publication of application: 24.05.2000
(73) Proprietor: Council of Scientific and Industrial Research, New Delhi 110 001 (IN)
(72) Inventor: Satyavathi, Bankupalli, Hyderabad 500 007 (IN); Patwari, Akash N.R., Hyderabad 500 007 (IN); Bhalerao, Uday T., Hyderabad 500 007 (IN)
(74) Representative: Towler, Philip Dean

(56) References cited:
- EP-A- 0 073 277
- EP-A- 0 148 009
- PATENT ABSTRACTS OF JAPAN vol. 011, no. 085 (C-410), 14 March 1987 & JP 61 238768 A (MITSUI PETROCHEM IND LTD), 24 October 1986

## Description

The present invention relates to a process for the preparation of alkylated aromatic amines. This invention particularly relates to an improved process for the selective preparation of ortho and N-alkylated aromatic amines. The present invention more particularly relates to an improved process for the preparation of 2,6-Dialkylamines such as 2,6-Diethylaniline or dimethylaniline and N-alkylated amines such as N-ethyl or N-methyl anilines. The process of the present invention involves the reaction of an aromatic amine and a primary or a secondary alcohol in the presence of a new catalyst containing attapulgite impregnated with a combination of iron oxide and the oxides selected from transition metals of the periodic table. The reaction is effected under moderate conditions of atmospheric pressure and low temperatures.

### BACKGROUND OF THE INVENTION:

Attapulgite used in the catalyst is a fibrous clay. The chemical analysis of attapulgite clay shows the presence of oxides such as SiO₂, Al₂O₃, MgO, Na₂O, Fe₂O₃ and water along with traces of nickel, chromium, zinc, copper, lead, tin, vanadium and silver and the composition of Attalpulgite in form of mole percent is as follows:

| | |
|---|---|
| SiO₂ | 55.03% |
| Al₂O₃ | 10.24% |
| Fe₂O₃ | 03.53% |
| MgO | 10.49% |
| K₂O | 00.47% |
| H₂O⁻ | 09.73% |
| H₂O₊ | 10.13% |

We have carried out extensive research in respect of the use of attapulgite for catalysing chemical reactions. Some of the other uses of attapulgite are listed here:-

It is used as a polymerisation catalyst, in refining vegatable oils and fats, as a carrier for granular and powdered agricultural chemicals (insecticides and herbicides), in petroleum refining, decolourising, neutralising, brightening and for desulphurisation.

It is also used as an oil base and water base foundry sand binders, latex paint thickener, gelling agent, polishing-suspending agent for abrasives and as a wax emulsion stabilizer.

We have observed during our research that attapulgite can be used as catalyst for alkylation reaction. On our continued work developing attapulgite as a catalyst for selective alkylation of aromatic amines we have observed that when attapulgite is impregnated with a combination of iron oxide and oxides selected from transition metals of the periodic table, the effect of alkylation is greatly enhanced. The catalysing activity of the resultant catalyst for the alkylation reaction, in particular for ortho and N-alkylation is found to be enchanced.

Alkylated aromatic amines are useful in a broad range of applications. Particularly ortho-alkylated aromatic amines are used as intermediates for producing dyes, Insecticides, resin stabilisers, rubber compounding ingredients and the like.

In the Japanese patent publication No. 47-24014/72 a method has been described in which aromatic amines such as aniline and the like are reacted with alkyl aluminium halide, followed by reaction of the resultant mixture with olefins. In Japanese patent No. 50-137934/75 a method has been described in which aromatic amines are reacted with lower olefins in the presence of a catalyst consisting of aluminium anilide and halogenated hydrocarbons. The above mentioned processes have advantages that the reaction activity and selectivity are high, but due to the involvement of very high temperatures and pressures there is a disadvantage that the reaction apparatus suitable for the conditions has to be used, and since a substantial amount of the catalyst is used, post treatment for removing the catalyst from the reaction mixture is troublesome.

Other prior art processes for producing ortho alkylated aromatic amines include methods like that proposed in U.S. Patent No. 2814646 in which an aromatic amine is reacted with olefins under heating in the presence of an aluminium anilide catalyst. The disadvantage of this process is that the reaction activity is low, although the selectivity in the product is high. In U.S. patent No. 4,351,958 a process for producing ortho-alkylated aromatic amines in which, an aromatic amine is reacted with olefins, has been described at a temperature in the range of 200-500°C with reaction pressure from 1-30 kg/cm² and a catalyst comprising of iron oxide as a main constituent. A variety of catalysts have been tried by this method and the maximum selectivity of 2,6-dialkylaniline is reported as 51.6% under optimum conditions.

N-Alkylated aromatic amines are useful in a broad range of applications. They are used as raw materials for synthesis of organic chemicals, and as intermediates for producing dyes, in agrochemical industries like fertilizers, as resin stabilisers, rubber compounding ingerdients and the like.

The vapour phase catalytic reaction of aniline and ethanol is the method of choice for the industrial production of N- ethylaniline. In European patent EP 39,061 a method has been described wherein aromatic amines are prepared treating olefins with ammonia, and a primary or a secondary amine in presence of Ruthenium or ferric compound dissolved in liquid phase solvent. The temperatures and pressures employed being 100-250°C and 1-12000 psig.

In Japanese patent 58,146,534 a method has been described in which N-alkylated anilines are prepared by alkylation of H₂NC₆H₅₋ₙXₙ with alcohols/ethers in gas phase in presence of S ion, Ti and Zr catalysts.

Other prior arts for producing N-ethylaniline include both liquid phase methods and vapour phase methods. These processes differ mostly in the type of catalyst used. The liquid phase processes produce N-ethylaniline with equal quantities of the diethylated products, and are still in vogue.

Several combinations of the catalyst have been reported, in particular alumina promoted with different metal oxides appears to have been widely employed. Zeolites have been used extensively as catalysts for the production of N-alkylated anilines. Bauxite is also proved to be a good catalyst for this reaction.

The main object of the present invention is to provide an improved process for the preparation of alkylated aromatic amines.

Another object of the present invention is to provide an improved process for the preparation of ortho-alkylated and N-alkylated aromatic amines. Yet another object of the present invention is to provide an improved process for the preparation of ortho-ethyl or ortho-methyl or N-ethyl or N-methyl amines. The invention is based on the findings that when attapulgite is impregnated with a combination of iron oxide and oxides selected from the transition metals of the periodic table, the activity of the resultant catalyst is enhanced for selective alkylation of the aromatic amines.

### DETAILED DESCRIPTION OF THE INVENTION:

Accordingly the present invention provides an improved process for the preparation of alkylated aromatic amines which comprises reacting an aromatic amine with an alcohol in the presence of a catalyst which comprises attapulgite together with iron oxide and one or more transition metal oxides. These catalysts are described in our EP-A-1002574 (agent's ref 33.69289), the disclosure of which is incorporated herein by reference. The reaction is generally conducted at atmospheric pressure at 300-400°C, and the product can be recovered by known methods.

The composition of the said catalyst as used in the process of our present invention comprises 1-75% of iron oxide, 1-10% of titanium metal oxide and the balance being attapulgate useful for the preparation of alkylated aromatic amines and is prepared by the process, which comprises impregnating attapulgite with a combination of iron oxide and oxides selected from transition metals of the periodic table, converting the resultant catalyst to the desired form, drying the catalyst and calcining by known methods.

The amine used in the process of present invention may be selected from aniline, o-toluidine, m-toluidine, p-toludine, p-ethylaniline, m-ethylaniline, O-ethylaniline, 2,3 Xylidine, N-ethylaniline, M-methylaniline.

The alcohol used in the process of present invention may be selected from methanol, ethanol, n-propanol, n-butyl alcohol, iso-butyl alcohol, iso-propyl alcohol. The amount of alcohol used may be in the range of 1-10 moles of alcohol based on 1 mole of the aromatic amine used. After completion of the reaction the ortho-alkylated aromatic amine maybe seperated from the reaction mixture by distillation.

The recovered alcohol and aromatic amine can be recycled. As side reactions N, N-dialkylamines maybe produced in quantities of 10-20%. These byproducts may be recycled to produce the desired alkylated amine under the same reaction conditions, thus in effect causing a quantitative conversion of the amine to the desired alkylated aromatic amines.

The metal oxides used for impregnation may be such as oxides of typical metallic elements such as aluminium oxide, silicon oxide, germanium dioxide, megnesium oxide, and oxides of the transition metals such as copper oxide, titanium oxide, zirconium oxide, chromium oxide and the like.

In our European patent application referred to above there is described and claimed a process for the preparation of the catalyst which is employed in the process of the present invention. Table 1 shows that the increase in percentage of Iron Oxide from 0-60% enhances the conversion of aniline and Table 2 shows the enhancement in the selectivity of the catalyst towards ring alkylation. The variation is shown in tables 1 and 2.

**TABLE- 1**

| % IRON OXIDE IN CATALYST VS CONVERSION AT DIFFERENT TEMPERATURES | | | | | |
|---|---|---|---|---|---|
| % Fe₂O₃ | X_{AO} (330°C) | X_{AO} (350°C) | X_{AO} (370°C) | X_{AO} (390°C) | X_{AO} (410°C) |
| 0 | 36.14 | 55.00 | 56.30 | 49.10 | 22.10 |
| 15 | 46.20 | 60.00 | 60.00 | 63.00 | 51.00 |
| 25 | 48.20 | 71.40 | 73.40 | 68.90 | 49.60 |
| 35 | 48.40 | 70.50 | 78.00 | 69.70 | 49.60 |
| 45 | 49.06 | 76.40 | 79.10 | 70.10 | 50.90 |
| 55 | 51.00 | 86.40 | 90.30 | 69.90 | 52.20 |
| 60 | 53.30 | 86.10 | 92.20 | 80.50 | 61.50 |
| 70 | 52.10 | 83.30 | 90.50 | 82.90 | 69.40 |
| * X_{AO} : OVERALL CONVERSION OF ANILINE | | | | | |

**TABLE-2**

| % IRON OXIDE IN CATALYST VS SELECTIVITY OF PRODUCTS | | | |
|---|---|---|---|
| % Fe₂O₃ | % Select MEA | % Select N,NDEA | % Select 2,6 DEA |
| 0 | 54.85 | 12.26 | 16.23 |
| 15 | 72.10 | 14.88 | 13.02 |
| 25 | 65.44 | 20.81 | 13.65 |
| 35 | 66.24 | 19.38 | 14.38 |
| 45 | 70.03 | 13.36 | 16.61 |
| 55 | 64.25 | 13.37 | 22.38 |
| 60 | 62.54 | 09.57 | 27.89 |
| 70 | 57.18 | 15.84 | 26.98 |
| * MEA:MONOETHYLANILINE | | | |
| N,N-DEA: N,N-diethylaniline | | | |
| 2,6-DEA: 2,6- diethylaniline | | | |

**TABLE-3**

| | | | | | | | |
|---|---|---|---|---|---|---|---|
| SHOWS THE EFFECT OF PROMOTERS ON THE CONVERSION AND SELECTIVITY OF PRODUCT COMPONENTS BY FIXING THE PERCENTAGE OF IRON OXIDE IN THE CATALYST | | | | | | | |

| TEMPERATURE VS SELECTIVITY OF MONOETHYLANILINE (USING DIFFERENT PROMOTERS) | | | | | | | |
|---|---|---|---|---|---|---|---|
| Temp (0C) | GeO₂ (1%) | Al₂O₃ (1%) | ZnO (1%) | MgO (1%) | Cr₂O₃ (1%) | SnO₂ (1%) | SnO₂ (2%) |
| 350 | 74.95 | 82.48 | 68.42 | 44.14 | 58.70 | 52.42 | 34.77 |
| 370 | 67.00 | 61.10 | 66.85 | 42.45 | 57.55 | 47.44 | 17.65 |
| 390 | 65.00 | 59.84 | 63.77 | 39.98 | 49.12 | 52.31 | 21.53 |
| 410 | 60.00 | 54.79 | 63.57 | 39.49 | 45.58 | 50.05 | 20.58 |

| TEMPERATURE VS SELECTIVITY OF N,N-DIETHYLANILINE (USING DIFFERENT PROMOTERS) | | | | | | | |
|---|---|---|---|---|---|---|---|
| Temp (0C) | GeO₂ (1%) | Al₂O₃ (1%) | ZnO (1%) | MgO (1%) | Cr₂O₃ (1%) | SnO₂ (1%) | SnO₂ (2%) |
| 350 | 16.00 | 11.73 | 15.75 | 09.84 | 18.80 | 05.24 | 09.68 |
| 370 | 17.00 | 28.85 | 14.84 | 09.82 | 18.85 | 05.88 | 10.24 |
| 390 | 18.65 | 29.55 | 16.56 | 10.00 | 20.56 | 06.86 | 12.33 |
| 410 | 17.24 | 30.48 | 16.11 | 08.86 | 19.32 | 06.47 | 10.10 |

| TEMPERATURE VS SELECTIVITY OF 2,6-DIETHYLANILINE (USING DIFFERENT PROMOTERS) | | | | | | | |
|---|---|---|---|---|---|---|---|
| Temp (0C) | GeO₂ (1%) | Al₂O₃ (1%) | ZnO (1%) | MgO (1%) | Cr₂O₃ (1%) | SnO₂ (1%) | SnO₂ (2%) |
| 350 | 09.05 | 05.84 | 15.83 | 09.64 | 23.03 | 42.32 | 55.53 |
| 370 | 16.00 | 10.05 | 18.31 | 09.95 | 23.35 | 46.68 | 72.11 |
| 390 | 16.35 | 10.61 | 19.67 | 09.90 | 30.32 | 40.83 | 66.14 |
| 410 | 22.76 | 14.82 | 20.32 | 11.00 | 35.10 | 43.48 | 46.88 |

The details of the invention is described in the following examples which are presented by way of illustration only and should not be construed to limit the scope of the present invention.

### EXAMPLES

### Example 1:

50 gmns of attapulgite containing iron oxide and tin oxide as catalyst is packed into a reactor and heated to 350°C. A liquid mixture of aniline and ethanol having a mole ratio of 1:5 is fed at a rate of 60 ml/hr whereby the reaction is effected. The product components so obtained are separated by distillation. The conversion of the amine is 65% and the yield of the ortho-alkylated aromatic amine is 44%.

### Example 2:

50 gms of attapulgite containing iron oxide and tin oxide as catalyst is packed into a reactor and heated to 370°C. A liquid mixture of aniline and ethanol having a mole ratio of 1:5 is fed at a rate of 60 ml/hr whereby the reaction is effected. The conversion of the amine is 69% and the yield of the ortho-alkylated aromatic amine is 50%.

### Example 3:

50 gms of attapulgite containing iron oxide and magnesium oxide as catalyst is packed into a reactor and heated at 370°C. A liquid mixture of aniline and ethanol having a mole ratio of 1:5 is fed at a rate of 60 ml/hr whereby the reaction is effected. The product components so obtained are separated by distillation. The conversion of the amine is 52% and the yield of the ortho-alkylated aromatic amine is 5%.

### Example 4:

50 gms of attapulgite containing iron oxide and Zinc oxide as catalyst is packed into a reactor and heated to 370°C. A liquid mixture of aniline and ethanol having a mole ratio of 1:5 is fed at a rate of 60 ml/hr whereby the reaction is effected. The product components are separated by distillation. The conversion of the amine is 58% and the yield of the ortho-alkylated aromatic amine is 11%.

### Example 5:

50 gms of attapulgite containing iron oxide and Chromium oxide as catalyst is packed into a reactor and heated to 370°C. A liquid mixture of aniline and ethanol having a mole ratio of 1:5 is fed at a rate of 60 ml/hr whereby the reaction is effected. The product components are separated by distillation. The conversion of the amine is 57% and the yield of the ortho-alkylated aromatic amine is 14%.

### Example 6:

50 gms of attapulgite impregnated with iron oxide and germanium dioxide as catalyst is packed into a reactor and heated to 375°C. A liquid mixture of aniline and ethanol having a mole ratio of 1:5 is fed kat rate of 60 ml/hr whereby the reaction is effected. The product components are separated by distillation. The conversion of aniline obtained is 87% and the yield of N-alkylanililne is 75%.

### Example 7:

50 gms of attapulgite impregnated with iron oxide and germanium dioxide as catalyst is packed into a reactor and heated to 350°C. A liquid mixture of aniline and ethanol having a mole ratio of 1:5 is fed at a rate of 60 ml/hr whereby the reaction is effected.

The product components are separated by distillation. The conversion of aniline obtained is 80% and the yield of N-alkylaniline is 64%.

### Example 8:

50 gms of attapulgite containing iron oxide and magnesium oxide as catalyst is packed into a reactor and heated to 370°C. A liquid mixture of aniline and ethanol having a mole ratio of 1:5 is fed at a rate of 60 ml/hr whereby the reaction is effected. The product components are separated by distillation. The conversion of the amine is 52% and the yield of the N-alkylated aromatic amine is 22%.

### Example 9:

50 gms of attapulgite impregnated with iron oxide and chromium oxide as catalyst is packed into reactor and heated to 375°C. A liquid mixture of aniline and ethanol having a mole ratio of 1:5 is fed at a rate of 60 ml/hr whereby the reaction is effected. The product components are separated by distillation. The conversion of aniline obtained is 57% and the yield of N-alkylaniline is 33%.

### Example 10:

50 gms of attapulgite impregnated with iron oxide and zinc oxide as catalyst ils packed into reactor and heated to 370°C. A liquid mixture of aniline and ethanol having a mole ratio of 1:5 is fed at rate of 60 ml/hr whereby the reactiom is effected. The product components are separated by distillation. The conversion of aniline obtained is 58% and the yield of N- alkylaniline is 38%..

## Claims

1. A process for the preparation of alkylated aromatic amines which comprises reacting an aromatic amine with an alcohol in the presence of a catalyst which comprises attapulgite together with iron oxide and one or more transition metal oxides.

2. A process according to claim 1 in which the alcohol is a C₁₋₆ alkanol.

3. A process according to claim 1 or claim 2 in which the aromatic portion of the amine is a phenyl group.

4. A process according to claim 1 which comprises reacting an aromatic amine selected from aniline, toludine, xylidine, N-methyl aniline, N-ethyl aniline, m-ethyl aniline, p-ethyl aniline, o-ethyl aniline with a primary or secondary alcohol selected from ethanol, methanol, isopropyl alcohol at atmospheric pressure and at a temperature in the range of 300-400°C and recovering the desired amine by conventional methods.

5. A process according to any preceding claim, wherein the amount of alcohol used is in the range of 1-10 moles based on 1 mole of the amine used.

6. A process according to any preceding claim, wherein the amount of amine used is in the range of 1-3 moles.

7. A process according to any preceding claim, wherein the alkylated aromatic amine produced is separated by distillation.

8. A process according to any preceding claim, wherein the by-product formed is re-cycled.

9. A process according to any preceding claim, in which the transition metal oxide is an oxide of copper, titanium, zirconium, chromium, germanium, tin, zinc, magnesium or aluminium.

10. A process according to any preceding claim in which the catalyst contains 1-75% by weight iron oxide and 1-10% by weight transition metal oxide.

11. A process according to any preceding claim in which the catalyst is prepared by impregnating or intimately mixing attapulgite with the iron oxide and the transition metal oxide, converting the resultant catalyst to the desired form, and drying and calcining the catalyst by known methods.

12. A process according to claim 11, wherein the drying of the catalyst is effected at a temperature in the range 90 to 100°C.

13. A process according to claim 11 or claim 12, wherein the catalyst is calcined at a temperature in the range of 400 to 450°C.

14. A process according to any of claims 1 to 8, wherein the catalyst used is attapulgite impregnated with iron oxide and a transition metal oxide selected from magnesium oxide, zinc oxide, chromium oxide and germanium oxide.

## Patentansprüche

1. Verfahren zum Präparieren von alkylierten aromatischen Aminen mit dem Schritt des zur Reaktion Bringens eines aromatischen Amins mit einem Alkohol im Beisein eines Katalysators, der Attapulgit zusammen mit Eisenoxyd und einem oder mehr Übergangsmetalloxyden umfaßt.

2. Verfahren nach Anspruch 1, bei dem der Alkohol ein C₁₋₆-Alkanol ist.

3. Verfahren nach Anspruch 1 oder 2, bei dem der aromatische Teil des Amins eine Phenylgruppe ist.

4. Verfahren nach Anspruch 1 mit den Schritten des zur Reaktion Bringens eines aromatischen Amins, das ausgewählt ist aus Anilin, Toludin, Xylidin, N-Methyl-Anilin, N-Ethyl-Anilin, m-Ethyl-Anilin, p-Ethyl-Anilin, o-Ethyl-Anilin mit einem primären oder sekundären Alkohol, der ausgewählt ist aus Ethanol, Methanol, Isopropyl-Alkohol bei atmosphärischem Druck und bei einer Temperatur in dem Bereich von 300-400°C und des Wiedergewinnens des gewünschten Amins durch herkömmliche Methoden.

5. Verfahren nach einem der vorstehenden Ansprüche, bei dem die verwendete Alkoholmenge in dem Bereich von 1-10 Mol basierend auf einem Mol des verwendeten Amins ist.

6. Verfahren nach einem der vorstehenden Ansprüche, bei dem die verwendete Aminmenge in dem Bereich von 1-3 Mol ist.

7. Verfahren nach einem der vorstehenden Ansprüche, bei dem das hergestellte alkylierte aromatische Amin durch Destillation getrennt wird.

8. Verfahren nach einem der vorstehenden Ansprüche, bei dem das gebildete Nebenprodukt wiederverwertet wird.

9. Verfahren nach einem der vorstehenden Ansprüche, bei dem das Übergangsmetalloxyd ein Kupferoxyd, Titanoxyd, Zirkoniumoxyd, Chromoxyd, Germaniumoxyd, Zinnoxyd, Zinkoxyd, Magnesiumoxyd oder Aluminiumoxyd ist.

10. Verfahren nach einem der vorstehenden Ansprüche, bei dem der Katalysator 1-75 Gewichtsprozent Eisenoxyd und 1-10 Gewichtsprozent Übergangsmetalloxyd enthält.

11. Verfahren nach einem der vorstehenden Ansprüche, bei dem der Katalysator durch Imprägnieren oder inniges Vermischen von Attapulgit mit dem Eisenoxyd und dem Übergangsmetalloxyd hergestellt wird und bei dem der resultierende Katalysator zu der gewünschten Form konvertiert wird und der Katalysator mit bekannten Methoden getrocknet und kalziniert wird.

12. Verfahren nach Anspruch 11, bei dem der Schritt des Trocknens des Katalysators bei einer Temperatur in dem Bereich von 90-100°C bewirkt wird.

13. Verfahren nach einem der Ansprüche 11 oder 12, bei dem der Katalysator bei einer Temperatur in dem Bereich 400-450°C kalziniert wird.

14. Verfahren nach einem der Ansprüche 1 bis 8, bei dem der verwendete Katalysator ein mit Eisenoxyd und einem Übergangsmetalloxyd, das ausgewählt ist aus Magnesiumoxyd, Zinkoxyd, Chromoxyd und Germaniumoxyd, imprägniertes Attapulgit ist.

## Revendications

1. Procédé de préparation d'aminés aromatiques alkylées qui comprend la réaction entre une amine aromatique et un alcool en présence d'un catalyseur qui comprend l'attapulgite avec de l'oxyde de fer et un ou plusieurs oxydes de métaux de transition.

2. Procédé selon la revendication 1, dans lequel l'alcool est un alcanol en C₁₋₆.

3. Procédé selon la revendication 1 ou la revendication 2, dans lequel la partie aromatique de l'amine est un groupement phényle.

4. Procédé selon la revendication 1 qui comprend la réaction entre une amine aromatique choisie parmi l'aniline, la toluidine, la xylidine, la N-méthyle aniline, la N-éthyle aniline, la m-éthyle aniline, la p-éthyle aniline, la o-éthyle aniline avec un alcool primaire ou secondaire choisi parmi l'éthanol, le méthanol, l'alcool isopropylique à pression atmosphérique et à température comprise entre 300 et 400°C et la récupération de l'amine désirée par des procédés conventionnels.

5. Procédé selon l'une quelconque des revendications précédentes, dans lequel la quantité d'alcool utilisée est comprise entre 1 à 10 moles pour 1 mole d'aminé utilisée.

6. Procédé selon l'une quelconque des revendications précédentes, dans lequel la quantité d'aminé utilisée est comprise entre 1 et 3 moles.

7. Procédé selon l'une quelconque des revendications précédentes, dans lequel l'amine aromatique alkylée produite est séparée par distillation.

8. Procédé selon l'une quelconque des revendications précédentes, dans lequel le produit dérivé formé est recyclé.

9. Procédé selon l'une quelconque des revendications précédentes, dans lequel l'oxyde de métal de transition est un oxyde de cuivre, de titane, de zirconium, de chrome, de germanium, d'étain, de zinc, de magnésium ou d'aluminium.

10. Procédé selon l'une quelconque des revendications précédentes, dans lequel le catalyseur contient 1 à 75% en poids d'oxyde de fer et 1 à 10% en poids d'oxyde de métal de transition.

11. Procédé selon l'une quelconque des revendications précédentes, dans lequel le catalyseur est préparé par l'imprégnation ou le mélange intime d'attapulgite avec l'oxyde de fer et l'oxyde de métal de transition, transformant le catalyseur résultant en la forme désirée, et le séchage et la calcination du catalyseur par des procédés connus.

12. Procédé selon la revendication 11, dans lequel le séchage du catalyseur est effectué à une température comprise entre 90 et 100°C.

13. Procédé selon la revendication 11 ou la revendication 12, dans lequel le catalyseur est calciné à une température comprise entre 400 et 450°C.

14. Procédé selon l'une quelconque des revendications 1 à 8, dans lequel le catalyseur utilisé est de l'attapulgite imprégnée d'oxyde de fer et d'un oxyde de métal de transition choisi parmi l'oxyde de magnésium, l'oxyde de zinc, l'oxyde de chrome et l'oxyde de germanium.
